(19) Europäisches Patentamt
European Patent Office
Office européen des brevets

(11) Veröffentlichungsnummer: **0 001 394**
**B1**

(12) **EUROPÄISCHE PATENTSCHRIFT**

(21) Anmeldenummer: **78100742.2**

(22) Anmeldetag: **24.08.78**

(51) Int. Cl.³: **C 07 D 233/74,**
C 07 D 235/02,
B 01 J 19/00

(54) **Verfahren zur kontinuierlichen Herstellung von Hydantoinderivaten und Vorrichtung zur Durchführung desselben.**

(30) Priorität: **03.10.77 DE 2744428**

(43) Veröffentlichungstag der Anmeldung:
**18.04.79 Patentblatt 79/8**

(45) Bekanntmachung des Hinweises auf die Patenterteilung:
**25.03.81 Patentblatt 81/12**

(84) Benannte Vertragsstaaten:
**BE CH DE FR GB NL**

(56) Entgegenhaltungen:
**DE - A - 1 695 646**
**DE - B - 1 166 201**
**FR - A - 2 207 916**

(73) Patentinhaber: **Lentia Gesellschaft mit beschränkter Haftung**
**Schwanthalerstrasse 39 Postfach 20 16 26**
**D-8000 München 2 (DE)**

(72) Erfinder: **Schweighofer, Johann, Dr.**
**Ghegastrasse 30**
**A-4020 Linz (AT)**

(72) Erfinder: **Schönbeck, Rupert, Dr.**
**Ortmayrstrasse 26**
**A-4060 Leonding (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 001 394 B1

# Verfahren zur kontinuierlichen Herstellung von Hydantoinderivaten und Vorrichtung zur Durchführung desselben

Die Erfindung bezieht sich auf ein Verfahren zur kontinuierlichen Herstellung von 5-substituierten Hydantoinen durch Umsetzung von Aldehyd- oder Ketoncyanhydrinen mit Ammoncarbonat in wäßrigem Medium bei erhöhter Temperatur, sowie auf die Vorrichtung zur Durchführung des Verfahrens.

Die Herstellung von in 5-Stellung substituierter Hydantoinen aus Cyanhydrinen und Ammoncarbonat ist bekannt. So erhält man beispielsweise nach einer allgemeinen Vorschrift (Organic Syntheses Coll.Vol.3, pg.323) durch mehrstündiges Erwärmen von 1 Mol Acetoncyanhydrin mit 1,3 Mol Ammoncarbonat auf 70 bis 90°C Dimethylhydantoin in 51 bis 56%iger Ausbeute, wobei nach Aufarbeitung aller Mutterlaugen weitere 30% eines ver unreinigten Produktes mit einem um 10°C verminderten Schmelzpunkt gewonnen werden.

Gemäß der DE—PS 566 094 erhält man durch mehrstündiges Erhitzen eines Ketons mit den äquivalenten Mengen an Ammoncarbonat und Alkalicyanid unter einem Kohlensäuredruck von 1,96 bis 9,8 bar 5-substituierte Hydantoine in quantitativer Ausbeute, die jedoch nicht rein sind, sodaß meist umkristallisiert werden muß.

In der US—PS 2 391 799 wird die Herstellung von Hydantoinen beschrieben, indem eine schwach phosphorsaure Lösung von Aceton und Blausäure während 104 Stunden in eine Ammoncarbonatlösung bei 62 bei 67°C unter gleichzeitigem Einleiten von $CO_2$ eingetragen und anschließend das gemäß der Reaktionsgleichung frei werdende Mol Ammoniak mit Direktdampf ausgeblasen und an die Umwelt abgegeben wird.

Im Gegensatz dazu wird gemäß dem Verfahren der DE—PS 11 66 201 diese Reaktion ohne Druck, jedoch in Gegenwart von überschüssigem Ammoniak durchgeführt, wobei eine Temperatur von unter 60°C zur Anwendung kommt. Dieses Verfahren kann auch kontinuierlich betrieben werden, es muß aber trotzdem ein Teil der Mutterlauge ausgetragen und gesondert aufgearbeitet werden.

Eine weitere Methode zur kontinuierlichen Herstellung von 5,5-Dialkylhydantoinen beschreibt die DE—PS 16 95 646, in der eine Mischung von Cyanhydrin und Wasser im Gegenstrom mit gasförmigen Kohlendioxyd und Ammoniak, beide in geringem Überschuß, in eine Rieselsäule geführt wird, in der durch eine Temperaturregelung von außen gewisse Temperaturbereiche in dem Kolonnenrohr eingehalten werden. Jedoch sind die in diesem Reaktionsrohr erzielbaren Ausbeuten nicht quantitativ, sondern leigen bei 85% und auch hier sind die anfallenden Mutterlaugen wegen der geringen Reinheit des darin enthaltenen Hydantoins gesondert aufzuarbeiten.

Überraschenderweise konnte nun ein kontinuierliches Verfahren gefunden werden, bei dem 1 Mol Cyanhydrin mit 1 Mol Ammoncarbonat so quantitativ und ohne Nebenreaktionen umgesetzt wird, daß eine völlige und mehrfache Kreislaufführung der Mutterlauge möglich ist, wobei bei einmaligem Durchgang quantitative Ausbeuten an den 5-substituierten Hydantoinen erzielt werden. Dies gelingt, indem man die Reaktionspartner in definierten Temperaturstufen im Gleichstrom umsetzt und das bei der Reaktion gebildete Ammoniak laufend ableitet und gemeinsam mit der Mutterlauge in die Reaktion zurückführt. Damit kann also jegliche Belastung des Abwassers oder der Atmosphäre vermieden werden.

Gegenstand der vorliegenden Erfindung ist demnach ein Verfahren zur kontinuierlichen Herstellung von 5-substituierten Hydantoinen der allgemeinen Formel

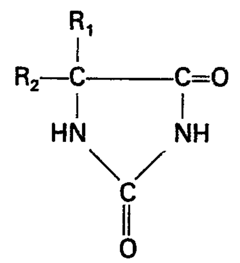
(I)

in der $R_1$ Wasserstoff oder Alkyl und $R_2$ Alkyl oder Alkenyl bedeuten oder $R_1$ und $R_2$ gemeinsam einen Alkylenrest mit 4 oder 5 C-Atomen in der Hauptkette darstellen, durch Umsetzung von Aldehyd- oder Ketoncyanhydrinen der allgemeinen Formel

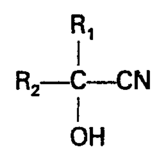
(II)

in der $R_1$ und $R_2$ wie oben definiert sind, mit Ammoncarbonat in wässerigem Medium bei erhöhter Temperatur, dadurch gekennzeichnet, daß man äquimolare Mengen der Reaktanten einsetzt, die Umsetzung im Gleichstrom in zwei oder mehreren voneinander getrennten Stufen mit steigendem Temperaturniveau innerhalb des Bereiches von 60 bis 100°C und zwar die erste Stufe bei einer Temperatur von 60 bis 85°C und die letzte Stufe bei einer Temperatur von 90 bis 100°C, durchführt und aus jeder Stufe das während der Reaktion gebildete Ammoniak laufend abzieht, worauf man aus der Reaktionslösung nach Kristallisation festes Hydantoin der Formel I in reiner Form ab-

trennt und die Mutterlauge nach Abtrennen von Reaktionswasser jedoch nach Einleitung des während der Reaktion gebildeten Ammoniaks und nach Zuführung von Kohlendioxyd und der zur Bildung von Ammoncarbonat in der dem umzusetszende Cyanhydrin der Formel II entsprechenden, zusätzlichen Menge an $NH_3$ in die Reaktion zurückführt. Besonders bevorzugt ist die Herstellung von Hydantoinen der obgenannten Formel I, worin $R_1$ Wasserstoff oder Alkyl mit maximal 5 Kohlenstoffatomen und $R_2$ Alkyl mit maximal 5 Kohlenstoffatomen, Vinyl, Allyl oder Propenyl bedeuten, welche als Zwischenprodukte zur Herstellung von Kunstharzen, Klebestoffen und Lacken dienen.

Für eine vorteilhafte Durchführung der Kristallisation hat es sich als günstig erwiesen, daß der Ammoniakgehalt der Kristallisationslösung auf unter 0,5%, bezogen auf Gesamtlösung, herabgesetzt wird.

Die Herabsetzung des Ammoniakgehaltes geschieht vorteilhafterweise entweder durch Durchblasen von Luft oder Inertgas während der Kristallisation oder durch Vakuumkristallisation, wobei im letzten Falle vorteilhafterweise aus der Gasphase des Vakummkristallisators nach Kondensation nur jene Wassermenge entnommen wird, die dem bei der Reaktion anfallenden einen Mol Wasser entspricht. Der Rest an kondensiertem Wasser kann in der Zentrifuge als Waschwasser verwendet und nach dem Waschen, mit der übrigen Mutterlauge vereint, der Reaktion weider zugeführt werden.

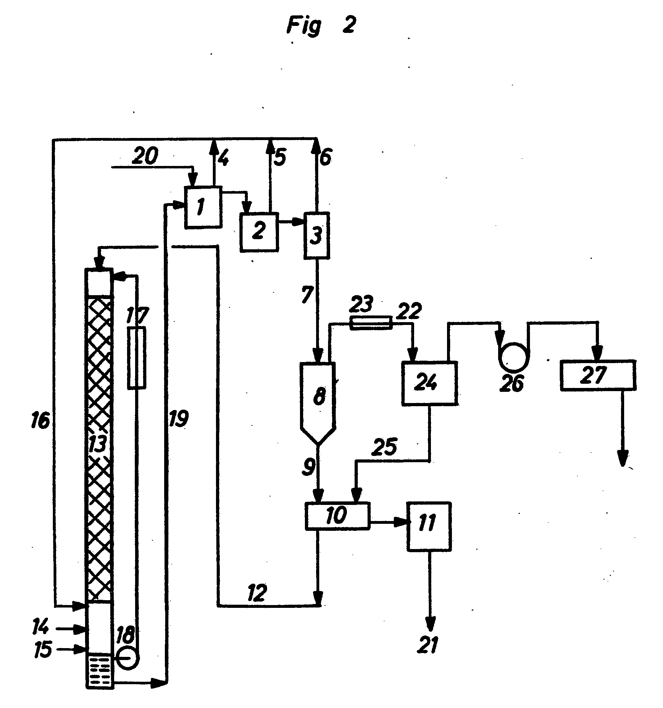

Ein weiterer Gegenstand der vorliegenden Erfindung ist eine Vorrichtung zur Durchführung des erfindungsgemäßen Verfahrens, die im Prinzip in Figur 1 wiedergegeben ist. In den Figuren 1 und 2 bedeuten 1, 2 und 3 die Reaktionsgefäße, die zu einer Kaskade hintereinandergeschaltet sind, 8 einen Kristallisator, 10 eine Zentrifuge, 11 einen Trockner, 7 und 9 einen Bodenablauf, 13 eine Absorptionskolonne, 17 und 23 einen Kühler, 18 eine Pumpe, 14, 15 und 16 Gaszuleitungsrohre, 4, 5, 6 und 22 Gasableitungsrohre, 19 eine Verbindungsleitung, 12 eine Mutterlaugenleitung, 24 einen Kondensator, 25 ein Kondensatableitungsrohr, 26 eine Vakuumpumpe, 27 eine Vorlage, 20 die Zuleitung für das Cyanhydrin und 21 die Produktentnahme.

Anhand von Figur 1 soll das Verfahren näher erläutert werden, ohne es jedoch darauf beschränken zu wollen. In ein Reaktionsgefäß 1 einer Reaktionskaskade werden kontinuierlich äquimolare Mengen von einem substituierten Cyanhydrin (über Leitung 20) und von Ammoncarbonat, letzteres gelöst in der nach Abzentrifugieren des Endproduktes anfallenden wässerigen Mutterlauge (über Leitung 19), eingeleitet und bei einer Temperatur von 60 bis 85°C gerührt. Kontinuierlich fließt die Reaktionslösung in ein zweites Reaktionsgefäß 2 der Reaktionskaskade, das auf einer um 5 bis 10°C höheren Temperatur gehalten ist, um von dort kontinuierlich in ein mit Dampf beheiztes, drittes Reaktionsgefäß 3, das eine Temperatur von 90 bis 100°C hat, zu gelangen. Alle 3 Gefäße sind mit Gasableitungsrohren 4, 5 und 6 versehen, aus denen über eine Sammelleitung 16 der bei der Reaktion frei werdende Ammoniak zusammen mit Wasser (dampf) in eine mit einem Kühler 17 in der Umpumpleitung versehene Absorptionskolonne 13 gelangt. Aus dem dritten Reaktionsgefäß 3 fließt die Reaktionsmischung über einen Bodenablauf 7 zum Kristallisator 8, dessen Bodenablauf 9 zur Zentrifuge 10 führt, in der das Endprodukt zentrifugiert, im Trockner 11 getrocknet und über die Produktentnahme 21 entnommen wird.

Die nach dem Zentrifugieren anfallende Mutterlauge gelangt über die Mutterlaugenleitung 12 in die mit einem Kühler 17 in der Umpumpleitung versehene Absorptionskolonne 13, in welche über dei Sammelleitung 16 Ammoniak und Wasser aus den Reaktionsgefäßen 1, 2 und 3 sowie über die Zuleitung 14 Kohlendioxyd und die Zuleitung 15 frisches Ammoniak zugeführt wird, und wird dort über den Kühler 17 mit Hilfe einer Pumpe 18 im Kreis geführt und auf etwa 30°C gehalten. Diese solcherart mit Ammoncarbonat aufkonzentrierte Mutterlauge wird über die Verbindungsleitung 19 in das Reaktionsgefäß 1 geführt, womit der Kreislauf geschlossen ist.

Bei der erfindungsgemäßen Reaktion entsteht 1 Mol Wasser, das, um Wasseranreicherungen zu vermeiden, aus dem System ausgeschleust wird. Besonders vorteilhaft ist es, wie in Figur 2 dargestellt ist, den Kristallisator 8 als Vakuumkristallisator auszuführen und die beim Kristallisieren anfallenden Dämpfe über ein Gasableitungsrohr 22 und einen Kühler 23 in einen Kondensator 24, an den eine Vakuumpumpe 26 und eine Vorlage 27 angeschlossen sind, zu leiten und zu kondensieren. Dabei kann die Kondensation so gelenkt werden, daß genau 1 Mol Wasser in der Vorlage 27 kondensiert, während der Hauptteil des Wassers aus dem Kondensator 24 über ein Kondensatableitungsrohr 25 in die Zentrifuge 10 geleitet und dort als Waschwasser verwendet wird.

## Beispiel 1

In ein Rührgefäß 1 aus Glas mit 1 l Inhalt, das durch einen Thermostaten auf 75°C gehalten wird, werden laufend pro Stunde eine Lösung von 192 g Ammoncarbonat und 166 g Dimethylhydantoin in 662 g Wasser aus der Absorptionskolonne 13 einerseits und 170 g Acetoncyanhydrin andererseits eingetragen. Die Lösung fließt in ein zweites Glasgefäß 2 von 1 l Inhalt über, in dem eine Temperatur von 85°C gehalten wird. Von hier fließt die Lösung in ein mit Dampf beheiztes und auf 95°C gehaltenes, drittes Reaktionsgefäß 3 von etwa 500 ml Inhalt. Aus allen 3 Gefäßen werden das austretende Ammoniak (26 g/h) und Wasser (200 g)

in die Absorptionskolonne 13 von 5 cm Durchmesser und 60 cm Höhe geleitet und dort kondensiert.

Aus dem dritten Reaktionsgefäß 3 fließt laufend die Reaktionsmischung (422 g Dimethylhydantoin, 534 g Wasser und 8 g Ammoniak pro Stunde) in einen Vakuumkristallisator 8 von etwa 2 l Inhalt. Im Kristallisator wird ein Vakuum von etwa 40 mbar mittels einer Pumpe 26 aufrecht gehalten. Von den abdestillierenden Produkten werden 76 g $H_2O$ und 4 g Ammoniak pro Stunde in dem Kondensator 24 kondensiert, während 24 g/h $H_2O$ in der Vorlage 27 nach der Vakuumpumpe 26 kondensiert werden. Diese Menge an Kondensat wird aus dem Kreislauf herausgenommen und verworfen.

Der Abfluß aus dem Vakuumkristallisator 8 läuft auf eine Zentrifuge 10, in der mit dem aus der Kondensation in 24 anfallenden, ammoniakhältigen Wasser gewaschen wird und 268 g feuchtes Dimethylhydantoin gewonnen werden, das nach der Trocknung 256 g reines Dimethylhydantoin mit Fp = 177°C gibt. Die Mutterlauge aus der Zentrifuge (498 g Wasser, 166 g Dimethylhydantoin und 8 g Ammoniak pro Stunde) wird kontinuierlich in die Absorptionskolonne 13 eingeführt, in der die Rückführung aus den Reaktoren 1, 2 und 3 und frisches $CO_2$ (88 g/h) und frisches $NH_3$ (34 g/h) kontinuierlich eingebracht werden. Die Mutterlauge wird mittels einer Pumpe 18 laufend über die Absorptionskolonne 13 gepumpt und mittels eines Kühlers 17 auf etwa 30°C gehalten. Aus der Absorptionskolonne 13 wird, wie schon oben angeführt, die Ammoncarbonatlauge in den Reaktor eingebracht.

Während 350 Stunden Fahrzeit blieb die Menge des gewonnen Dimethylhydantoins gleich und das Produkt war gleichmäßig rein. Eine Anreicherung von Nebenprodukten in der Mutterlauge konnte nicht festgestellt werden.

Beispiel 2

In einer Apparatur gemäß Figur 2 werden laufend pro Stunde eine Lösung von 192 g (2 Mole) Ammoncarbonat und 180 g Methylhydantoin in 662 g $H_2O$ sowie 144 g (2 Mole) Acetaldehydcyanhydrin analog Beispiel 1 umgesetzt und aufgearbeitet, wobei 226 g Methylhydantoin mit einem Schmelzpunkt von 147°C erhalten werden.

Beispiel 3

In einer Apparatur gemäß Figur 1 werden laufend pro Stunde eine Lösung von 192 g (2 Mole) Ammoncarbonat und 50 g Isopropylhydantoin in 662 g $H_2O$ sowie 198 g (2 Mole) Isobutyraldehydcyanhydrin umgesetzt. Die ausreagierte Lösung gelangt vom letzten Gefäß 3 des Kaskadenreaktors in den Kristallisator 8, in dem mittels Durchblasen von Luft auf 35°C abgekühlt und gleichzeitig der $NH_3$-Gehalt auf unter 0,5% abgesenkt wird, und von dort in die Zentrifuge 10. Aus dem Trockner 11 werden 281 g Isopropylhydantoin mit einem Schmelzpunkt von 152°C gewonnen. Die anfallenden Mutterlaugen werden wie in den Beispielen 1 und 2 in die Absorptionskolonne 13 rückgeführt, dort mit Ammoniak und Kohlendioxyd aufkonzentriert und dem ersten Gefäß 1 des Kaskadenreaktors zusammen mit frischem Isobutyraldehydcyanhydrin als Einsatzlösung zugeführt.

## Patentansprüche

1. Verfahren zur kontinuierlichen Herstellung von 5-substituierten Hydantoinen der allgemeinen Formel

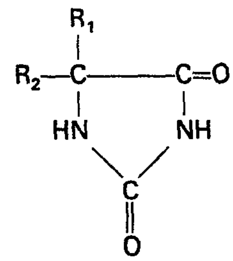

(I)

in der $R_1$ Wasserstoff oder Alkyl und $R_2$ Alkyl oder Alkenyl bedeuten oder $R_1$ und $R_2$ gemeinsam einen Alkylenrest mit 4 oder 5 C-Atomen in der Hauptkette darstellen, durch Umsetzung von Aldehyd- oder Ketoncyanhydrinen der allgemeinen Formel

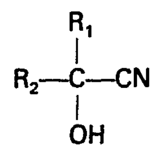

(II)

in der $R_1$ und $R_2$ wie oben definiert sind, mit Ammoncarbonat in wässerigem Medium bei erhöhter Temperatur, dadurch gekennzeichnet, daß man äquimolare Mengen der Reaktanten einsetzt, die Umsetzung im Gleichstrom in zwei oder mehreren voneinander getrennten Stufen mit steigendem Temperaturniveau innerhalb des Bereiches von 60 bis 100°C und zwar die erste Stufe bei einer Temperatur von 60 bis 85°C und die letzte Stufe bei einer Temperatur von 90 bis 100°C, durchführt und aus jeder Stufe das während der Reaktion gebildete Ammoniak laufend abzieht, worauf man aus der Reaktionslösung nach Kristallisation festes Hydantoin der Formel I in reiner Form abtrennt und die Mutterlauge nach Abtrennen von Reaktionswasser jedoch nach Einleitung des während der Reaktion gebildeten Ammoniaks und nach Zuführung von Kohlendioxyd und der zur Bildung von Ammoncarbonat in der dem um-

zusetzenden Cyanhydrin der Formel II entsprechenden, zusätzlichen Menge an $NH_3$ in die Reaktion zurückführt.

2. Verfahren nach Anspruch 1, dadurch gekennzeichnet, daß der Ammoniakgehalt der Reaktionslösung in der Kristallisation auf unter 0,5%, bezogen auf die Gesamtlösung, herabgesetzt wird.

3. Verfahren nach den Ansprüchen 1 und 2, dadurch gekennzeichnet, daß man die Kristallisation im Vakuum durchführt und die im Destillat anfallende, wässerige Ammoniaklösung abzieht und, gegebenenfalls nach Einsatz als Waschlösung für das Kristallisat, mit der Mutterlauge der Kristallisation vereinigt.

4. Verfahren nach Anspruch 2, dadurch gekennzeichnet, daß die Kristallisation bei Normaldruck unter gleichzeitigem Durchleiten von Luft oder Inertgasen durchgeführt wird.

5. Vorrichtung zur Durchführung eines Verfahrens zur kontinuierlichen Herstellung von 5-substituierten Hydantoinen der allgemeinen Formel

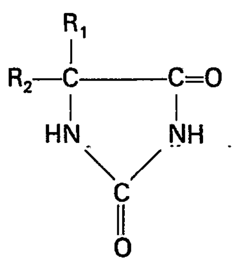

(I)

in der $R_1$ Wasserstoff oder Alkyl und $R_2$ Alkyl oder Alkenyl bedeuten oder $R_1$ und $R_2$ gemeinsam einen Alkylenrest mit 4 oder 5 C-Atomen in der Hauptkette darstellen, durch Umsetzung von äquimolaren Mengen von Aldehyd- oder Ketoncyanhydrinen, der allgemeinen Formel

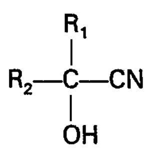

(II)

in der $R_1$ und $R_2$ wie oben definiert sind, und Ammoncarbonat in wäßrigem Medium gekennzeichnet durch

a) einen Kaskadenreaktor mit den drei Reaktorgefäßen (1,2,3) samt je einem Gasableitungsrohr (4,5,6) und der Zuleitung für Cyanhydrin (20) zum Reaktorgefäß (1),

b) einen Kristallisator (8), der mit dem Bodenablauf (7) des dritten Reaktogefäßes (3) verbunden ist,

c) eine Zentrifuge (10), die mit dem Bodenablauf (9) des Kristallisators (8) verbunden ist, und

d) eine mit Umwälzpumpe (18) und Kühler (17) augestattete Absorptionskolonne (13), die am Kopf mit der Mutterlaugenlösung (12) von der Zentrifuge (10) verbunden ist, am Fuß Zuführungsleitungen (14) für Kohlendioxyd, (15) für frisches Ammoniak und (16) für rückgeführtes Ammoniak samt Wasserdampf aufweist und von deren Bodenablaß die den Kreislauf schließende Leitung (19) zum Reaktorgefäß (1) führt (Fig.1).

6. Vorrichtung zur Durchführung eines Verfahrens nach Anspruch 5, dadurch gekennzeichnet, daß der Kristallisator (8) als Vakuumkristallisator ausgeführt ist, von welchem über das Gasableitungsrohr (22), einen Kühler (23) und den Kondensator (24) das Kondensatableitungsrohr (25) zur Zentrifuge (10) führt, wobei zur Ausschleusung des Reaktionswassers die Vorlage (27) der Vakuumpumpe (26) nachgeschaltet ist (Fig.2).

**Revendications**

1. Procédé pour la production en continu d'hydantoïnes substituées en position 5, de formule générale:

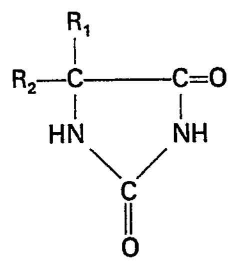

(I)

dan laquelle $R_1$ est de l'hydrogène ou un groupe alcoyle et $R_2$ est un groupe alcoyle ou alcényle, ou bien $R_1$ et $R_2$ forment conjointement un groupe alcoylène comprenant 4 ou 5 atomes de carbone dans la chaîne principale, par réaction de cyanhydrines dérivées d'aldéhydes ou de cétones de formule générale:—

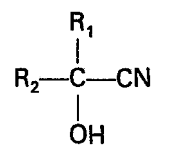

(II)

dans laquelle $R_1$ et $R_2$ ont la même signification que précédemment, avec du carbonate d'ammonium, en milieu aqueux à température élevée, caractérisé en ce qu'on utilise des quantités équimolaires des réactifs, on effectue la réaction en courant régulier dans deux ou plusieurs étages séparés l'un de l'autre, avec des niveaux de températures croissant dans une gamme allant de 60 à 100°C, le premier étage étant effectué à une température de 60 à 85°C et le dernier étage à une température de 90 à 100°C, et on prélève à chaque étage de façon continue l'ammoniac formé lors de la réaction, après quoi on sépare de la solution réactionnelle, après cristallisation,

l'hydantoïne solide de formule (I) sous forme pure, et on renvoie la liqueur-mère à la réaction après séparation de l'eau de réaction, mais toutefois après introduction de l'ammoniac formé pendant la réaction et après admission d'anhydride carbonique et de $NH_3$ pour la formation de carbonate d'ammonium, selon une quantité additionnelle correspondant à la cyanhydrine de formule II devant réagir.

2. Procédé suivant la revendication 1, caractérisé en ce que la teneur en ammoniac de la solution réactionnelle au cours de la cristallisation est réduite à une valeur inférieure à 0,5% calculée sur la base de la quantité totale de solution.

3. Procédé suivant les revendications 1 et 2, caractérisé en ce qu'on effectue la cristallisation sous vide et on prélève la solution aqueuse d'ammoniac présente dans le distillat et, éventuellement après utilisation comme solution de lavage pour le produit de cristallisation, on réunit à la liqueur-mère résultant de la cristallisation.

4. Procédé suivant la revendication 2, caractérisé en ce qu'on effectue la cristallisation à la pression normale, avec passage simultané d'air ou de gaz inertes.

5. Dispositif pour la mise en oeuvre d'un procédé de préparation en continu d'hydantoïnes substituées en position 5 de formule générale:—

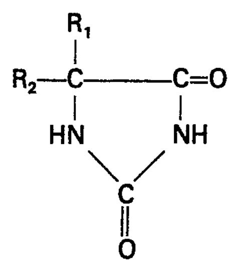
(I)

dans laquelle $R_1$ est de l'hydrogène ou un groupe alcoyle et $R_2$ est un groupe alcoyle ou alcanyle, ou bien $R_1$ et $R_2$ forment conjointement un radical alcoylène ayant 4 ou 5 atomes de carbone dans la chaîne principale, par réaction de quantités équimolaires de cyanhydrines dérivées d'aldéhydes ou cétones de formule générale:—

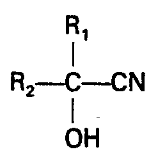
(II)

dans laquelle $R_1$ et $R_2$ ont la même signification que précédemment, et de carbonate d'ammonium, en milieu aqueux, caractérisé en ce qu'il comprend:—

a) un réacteur en cascade comprenant trois récipients formant réacteurs (1,2,3), ainsi que pour chacun une tubulure d'évacuation des gaz (4,5,6), et un conduit d'admission pour la cyanhydrine (20) introduite dans le réacteur (1).

b) un appareil de cristallisation (8) qui est relié à l'orifice d'écoulement inférieur (7) du troisième réacteur (3),

c) un centrifugeur (10) qui est relié à l'orifice d'écoulement inférieur (9) de l'appareil de cristallisation (8), et

d) une colonne d'absorption (13) équipée d'une pompe de circulation (18) et d'un dispositif de refroidissement (17), cette colonne étant relieé à son sommet à la solution de liqueur-mère (12) du centrifugeur (10) et comprenant à sa base des conduits d'admission (14) pour l'anhydride carbonique, (15) pour l'ammoniac frais et (16) pour l'ammoniac recyclé conjointement à la vapeur d'eau, et à partir de l'orifice d'écoulement inférieur de laquelle le conduit (19) bouclant le circuit aboutit au réacteur (1) (Fig.1).

6. Dispositif pour la mise en oeuvre d'un procédé suivant la revendication 5, caractérisé en ce que l'appareil de cristallisation (8) se présente sous la forme d'un cristallisoir sous vide, à partir duquel le conduit d'évacuation du condensat (25) aboutit au centrifugeur (10) par l'intermédiaire de la tubulure d'évacuation des gaz (22), d'un dispositif de refroidissement (23) et du condenseur (24), le collecteur (27) étant monté en aval de la pompe à vide (26) pour la séparation de l'eau de réaction par effet de sas (Fig.2).

**Claims**

1. Process for the continuous preparation of 5-substituted hydantoins of the general formula

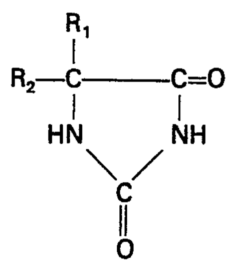
(I)

in which $R_1$ denotes hydrogen or alkyl and $R_2$ denotes alkyl or alkenyl, or $R_1$ and $R_2$ conjointly represent an alkylene radical with 4 or 5 C atoms in the main chain, by reaction of aldehyde-cyanohydrins or ketone-cyanohydrins of the general formula

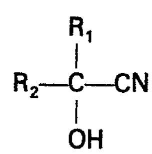
(II)

in which $R_1$ and $R_2$ are defined as above, with ammonium carbonate in an aqueous medium at an elevated temperature, characterised in that equimolar amounts of the reactants are employed, the reaction is carried out in cocurrent in two or more separate stages with a rising temperature level within the range from 60 to 100°C, in particular with the first stage at a temperature of 60 to 85°C and the last stage at a temperature of 90 to 100°C, and the ammonia formed during the reaction is taken off continuously from each stage, following which solid hydantoin of the formula I is separated off, in a pure form, from the reaction solution after crystallisation, and the mother liquor is recycled to the reaction after separating off the water of reaction but after introducing the ammonia formed during the reaction and after feeding in carbon dioxide and an additional amount of $NH_3$ so that ammonium carbonate is formed in an amount corresponding to the cyanohydrin of the formula II which is to be reacted.

2. Process according to Claim 1, characterised in that the ammonia content of the reaction solution is lowered, in the crystallisation stage, to less than 0.5%, relative to the total solution.

3. Process according to Claims 1 and 2, characterised in that the crystallisation is carried out in vacuo and the aqueous ammonia solution obtained in the distillate is taken off and is combined, optionally after having been employed as a wash solution for the crystals, with the mother liquor from the crystallisation.

4. Process according to Claim 2, characterised in that the crystallisation is carried out under normal pressure, whilst at the same time passing air or inert gases through the mixture.

5. Apparatus for carrying out a process for the continuous preparation of 5-substituted hydantoins of the general formula

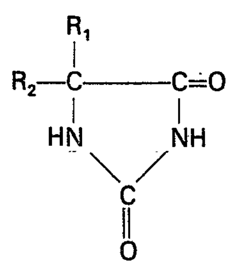

(I)

in which $R_1$ denotes hydrogen or alkyl and $R_2$ denotes alkyl or alkenyl or $R_1$ and $R_2$ conjointly represent an alkylene radical with 4 or 5 C atoms in the main chain, by reaction of equimolar amounts of aldehyde-cyanohydrins or ketone-cyanohydrins of the general formula

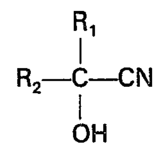

(II)

in which $R_1$ and $R_2$ are as defined above, and ammonium carbonate in an aqueous medium, characterised by

a) a cascade reactor with the three reactor vessels (1,2,3) together with a gas discharge pipe (4,5,6) for each vessel and the feed line for cyanohydrin (20) to the reactor vessel (1).

b) a crystalliser (8) which is connected to the bottom outlet (7) of the third reactor vessel (3),

c) a centrifuge (10) which is connected to the bottom outlet (9) of the crystalliser (8) and

d) an absorption column (13), which is equipped with a circulating pump (18) and cooler (17), is connected at the top to the mother liquor solution (12) from the centrifuge (10) and possesses, at the bottom, feed lines (14) for carbon dioxide, (15) for fresh ammonia and (16) for recycled ammonia together with water vapour, the line (19) which completes the circuit leading from the bottom outlet of the column to the reactor vessel (1) (Figure 1).

6. Apparatus for carrying out a process according to Claim 5, characterised in that the crystalliser (8) is constructed as a vacuum crystalliser, from which the condensate discharge pipe (25) leads, via the gas discharge pipe (22), a cooler (23) and the condenser (24) to the centrifuge (10), the receiver (27) being provided downstream of the vacuum pump (26) for discharging the water of reaction (Figure 2).

Fig 1

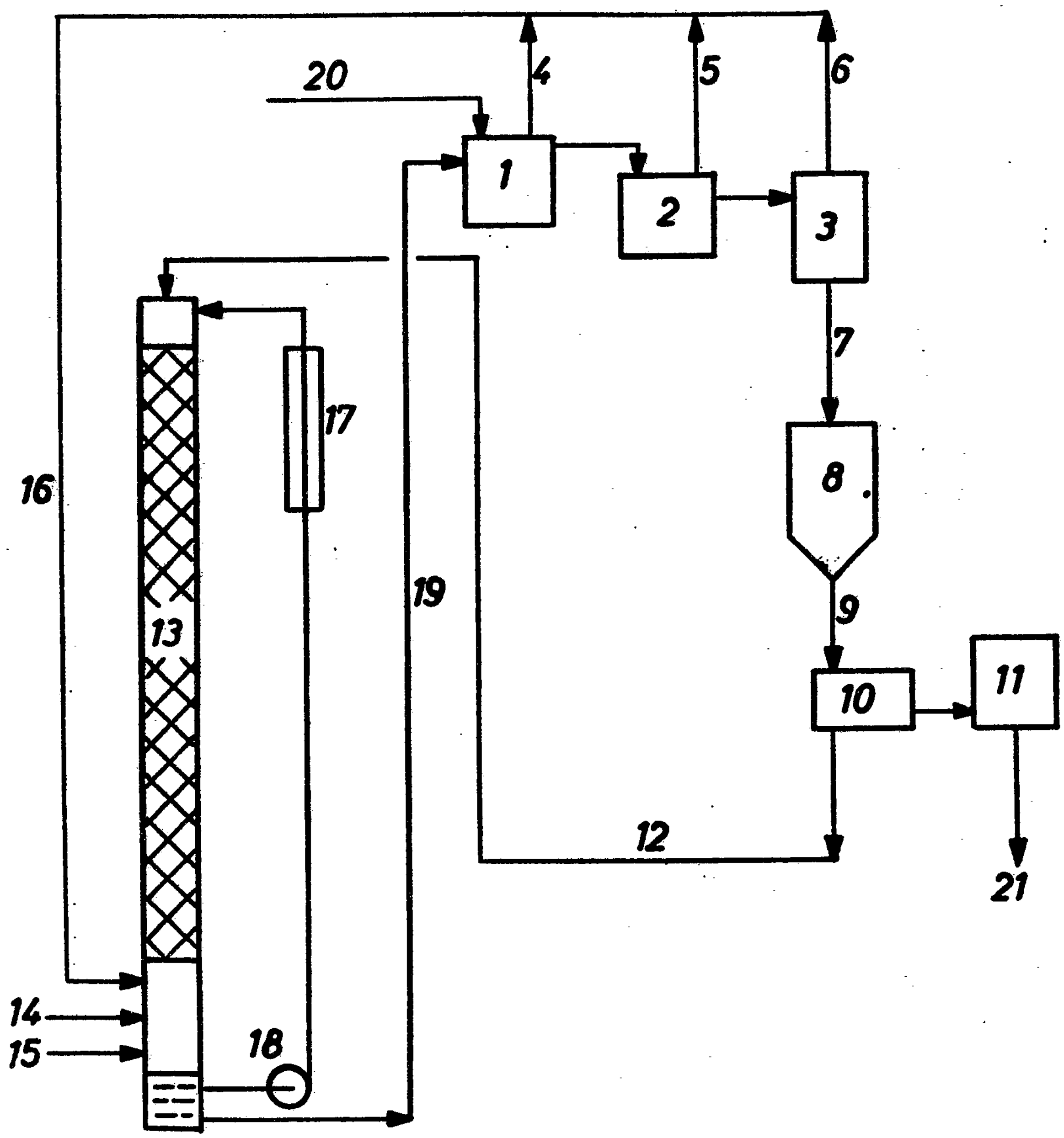

Fig 2